# EUROPEAN PATENT APPLICATION

(11) **EP 4 201 199 A1**
(43) Date of publication of application: **28.06.2023**
(21) Application number: 21217249.8
(22) Date of filing: 23.12.2021
(51) Int. Cl.: A01H 1/00, C12N 1/12

(54) **METHODS OF SELECTING MICROALGAE & PRODUCTS THEREOF**

(71) Applicant: Alver World SA, 1566 Saint-Aubin (CH)
(72) Inventor: URAN, Mine, 1803 Chardonne (CH); MOROSINOTTO, Tomas, 25015 Desenzano del Garda BS (IT); BELLAN, Alessandra, 35048 Stanghella PD (IT)
(74) Representative: reuteler & cie SA

(57) **Abstract**

The present invention relates to a method of selecting microalgae with reduced pigment content, in particular *Chrorella* strains such as *C*. *vulgaris* and resulting new microalgae strains and products therefrom.

## Description

### Field of the invention

The present invention relates to a method for selecting microalgae with reduced pigment content, in particular *Chlorella* strains such as C. *vulgaris* and resulting new microalgae strains and products therefrom.

### Background

Society is becoming increasingly conscious of sustainable nutrition as the world's population keeps rising *(*FAO, 2017: The future of food and agriculture. Trends and challenges. Rome: Food and Agriculture Organization of the United States*).* The unpredictability of the future of food production with respect to water and land usage, in addition to the changing in consumer preferences, brings the need for additional food sources having a high nutritional quality *(*van Huis et al., 2014, Edible insects - Future prospects for food and feed security. Rome: FAO*).*

As the negative impact of animal rearing on the environment such as shortage of water, stripping the land of nutrients, and increase of pollution (antibiotics for livestock) became fully aware in our society, a more environmentally friendly lifestyle has been targeted by vegetarian and vegan population. Thus, there is a growing need for especially protein-rich foods since vegans do not have access to animal proteins (e.g., eggs) *(van Huis et al., 2014, supra; FAO, 2017, supra;* Henchion et al 2017; Foods,6 (7). DOI: 10.3390foods6070053*).*

Current protein sources are not fulfilling the growing demand for low environmental impact and high protein sources *(Henchion et al, 2017, supra).*

For decades, microalgae have been recognized as superfood for being both healthy and sustainable *(*Vrenna et al., 2021, Sustainability 13 (5), p. 2848. DOI: 10.3390/su13052848*;* Kusmayadi et al., 2021, Chemosphere 271, p. 129800. DOI: 10.1016/j.chemosphere.2021.129800*;* Koyande et al. 2019, Food Science and Human Wellness, 8 (1), 16-24. DOI: 10.1016/j.fshw.2019.03.001*).* Microalgae-based proteins require less land and water compared to animal-based proteins and for their cultivation, non-arable land is used *(*Caporgno et al., 2018, Frontiers in nutrition, 5, p. 58. DOI: 10.3389/fnut.2018.00058*).* Today, microalgae are considered as promising sustainable alternative protein sources for the future. Microalgae are rich sources of protein (more than 50%) and have well-balanced amino acid profiles regarding the recommendations of the WHO/FAO/UNU *(*Vieira et al., 2020, New and Future Developments in Microbial Biotechnology and Bioengineering, 54: Elsevier, 19-30*;* FAO/WHO/UNU Expert Consultation on Energy and Protein Requirements. AMINO ACID SCORING PATTERNS (1981*). With assistance of A. Harper. Rome. Available online at http:llwww.fao.org*/*3*/*M3013E*/*M3013E00.htm, checked on 28*/*06*/*2021).*

Several microalgae species are widely used as nutritional supplements in tablet, capsule, or powdered form due to their taste, color, smell, and cost *(*Lafarga, 2019, Algal Research, 41, 101566. DOI: 10.1016/j.algal.2019.101566*).* The bacterium *Spirulina* and the microalgae *Chlorella* are two of the commercially important species on the market today *(*Nilesh Hemantkumar et al., 2020 Milada Vitová (Ed.): Microalgae - From Physiology to Application: IntechOpen*),* especially due to their high protein contents *(*Andrade, 2018, MOJFPT 6 (1). DOI: 10.15406/mojfpt.2018.06.00144*).* Microalgae contain also other healthy compounds besides proteins that can be used for several applications *(*Barkia et al., 2019 Marine drugs, 17 (5). DOI: 10.3390/md17050304*;* Milledge, 2011, Rev. Environ. Sci. Biotechnol., 10.1007/s11157-010-9214-7*).* Even if large-scale cultivation of *Chlorella vulgaris* is a consequence of the interest on the protein fraction accumulated and the content of essential amino acids, other beneficial nutrients such as vitamins (B-complex and ascorbic acid), minerals (potassium, sodium, magnesium, iron, and calcium), and pigments (β-carotene and chlorophyll) can be exploited using these microalgae *(*Becker, 2007, Biotechnology advances, 25 (2), 207 210. DOI: 10.1016/j.biotechadv.2006.11.002*;* Rodriguez-Garcia et al., 2008, Food Chem., 108, 1023-1026*).*

On the other hand, as obtained from a microalga, *Chlorella* products have a green color, a bitter taste and a strong smell because of their chlorophyll content. Their application in the food industry is limited, especially in those products where the color of the microalgae cannot be masked with other ingredients, like in dairy products. In some food products, microalgae are also used as natural food colorants thanks to their pigments *(Becker, 2007, supra;* Caporgno et al., 2018, Frontiers in nutrition 5, 58. DOI: 10. 3389/fnut.2018. 00058*). C. vulgaris* is growing fast and therefore cultivation techniques can be tailored to optimize protein content and biomass yield *(*Fransiscus et al., 2017, AIP conference Proceedings, 1840, 030005*).* The reason is that different growth conditions cause C. *vulgaris* to modify the yield of biomass concerning protein, lipid, carbohydrate, and/or pigment content *(*Ibrahim et al., 2020, AJVS 65, (1), 16. DOI: 10.5455/ajvs.94847*).* Depending on the strain, C. *vulgaris* can be grown autotrophically, heterotrophically or mixotrophically *(*Iwamoto,2003 Amos Richmond (Ed.): Handbook of Microalgal Culture. Oxford, UK: Blackwell Publishing Ltd, 253-263*,* Fatemeh et al., 2016, Journal of Applied Sciences and Environmental Management, 20 (1), 133. DOI: 10.43141jasem.v20i1.16*).* Autotrophically, C. *vulgaris* are usually grown in open pond systems (photosynthetically) for large-scale biomass production (autotrophic conditions) as open pond systems are cheap in maintenance *(Ibrahim et al., 2020, supra.).* Heterotrophic (dark) growth conditions are found to reduce the chlorophyll content *(*Khan et al., 2016, Heterotrophic Growth of Micro Algae. In J. Liu, Z. Sun, H. Gerken (Eds.): Recent Advances in Microalgal Biotechnology*)* which can be a promising solution regarding the customer acceptance *(*Boeing et al.,2015, 12th European Nutrition Conference (FENS), Berlin, Germany, October 20-23, 2015: Abstracts. In Annals of nutrition & metabolism 67 Suppl 1, 1-601. DOI: 10.1159/000440895*).* The heterotrophic growth technique is a process in which microalgae are grown on an organic carbon source, such as glucose, glutamate, and glycerol as well as on additional nutrients *(*Brennan et al., 2010 Renewable and Sustainable Energy Reviews, 14 (2), 557-577. DOI: 10.1016/j.rser.2009.10.009*;* Nikodinovic-Runic et al., 2013 Advances in applied microbiology, 84, 139-200. DOI: 10.1016/B978-0-12-407673-0.00004-7*).* Heterotrophic growth techniques instead of open pond systems allow to achieve a faster growth, higher dry biomass productivity, high yield of different components like proteins or lipids *(Ibrahim et al., 2020, supra.;* Hu et al., 2018, Biotechnology Advances 36, 54-67*).* Chlorophyll content is also reduced in heterotrophic conditions due to the absence of light *(Khan et al., 2016, supra).* Microalgae cells first grown heterotrophically significantly accumulate chlorophyll when later on exposed to light *(*Ogbonna et al., 1997, Journal of Applied Phycology, 9, 359-366, doi: 10.1023/A:1007981930676*).*

By using microalgae as a new food and feed platform, there is an opportunity to increase the supply of essential products to address global demands in a more efficient and environmentally sustainable way. It is therefore needed to enhance the nutritional content, productivity and organoleptic profile of algae to support the development of this new form of crop. Traditionally, screening microalgae for potential heterotrophy has relied on growth assays to assess their ability to metabolise organic carbon in the absence of light *(*Hee et al., 2019, Sci. Rep. 9, 19383). While these assays are informative, they can often imply laborious extensive laboratory equipment. Several platforms have been developed for the selection of heterotrophic microalgae having interesting properties *(Jareonsin et al., 2021, 9: 628597;* Sutherland et al., 2021, New BIOTECHNOLOGY, 65 (2021) 61-68*).*

However, there is still a need to develop new methods for the rapid assessment of microalgal heterotrophic metabolism allowing a cost-effective screening of algae with interesting physiological profile since organoleptic factors such as color, taste and smell can be decisive for the acceptability of foods supplemented with microalgae.

Consequently, there is a need for cost-effective innovative techniques for the selection of interesting of heterotrophic algae strains, in particular from C. *vulgaris species* and for the provision with new strains with lower chlorophyll content with suitable taste for food applications and adapted to large scale production.

### Summary of the Invention

An object of this invention is to provide a method for selecting microalgae strains belonging to green algae and blue-green algae genus, in particular *Chlorella* strains such as C. *vulgaris* which present a low chlorophyll content (e.g. <5%).

It is advantageous to provide a method which allows the selection of microalgae strains which present a low chlorophyll content not directly on their chlorophyll content but for their inability to grow photo-autotrophically.

It is advantageous to provide a method which allows the selection of microalgae strains presenting a high biomass productivity (e.g. high growth rate) under heterotrophic growth conditions.

It is advantageous to provide a method which allows the production of microalgae suitable for human consumption as food ingredient and/or a food supplement and has a reduced chlorophyll content.

It is advantageous to provide a method which allows the production of microalgae suitable for human consumption without exogenous DNA addition.

It is advantageous to provide a method which allows the production of microalgae with a constitutive reduction in chlorophyll in absence of specific treatment (ex. nutrient depletion) to modulate chlorophyll content.

Objects of this invention have been achieved by providing a method according to claim 1 and products thereof.

Objects of this invention have been achieved by providing a strain according to claim 9.

Objects of this invention have been achieved by providing a microorganism which is, or has the identifying characteristics of, a strain of C. *vulgaris* according to claim 10.

Objects of this invention have been achieved by providing a use of a strain according to claim 11.

Objects of this invention have been achieved by providing a food product according to claim 15.

Disclosed herein, according to a first aspect of the invention, is a method for the selection of microalgae, wherein said method comprises the following steps:
**a)** Providing a plurality of microalgae strains belonging to the same green algae and blue-green algae genus to a microalgae heterotrophic culture solid medium;
**b)** Growing said plurality of strains in said heterotrophic culture medium under heterotrophic culture conditions for about 10 to about 60 days;
**c)** Selecting a first sub-set of strains based on their reduced coloration;
**d)** Subjecting said first sub-set of strains with a reduced coloration to a growing step in microalgae heterotrophic culture medium under heterotrophic culture conditions for about 1 to 10 days;
**e)** Selecting a second sub-set from said first sub-set of strains based on a heterotrophic growth rate not lower than the 80% of the growth rate of the parental strain in the same conditions;
**f)** Growing said second sub-set under autotrophic culture medium under autotrophic culture conditions for about 1 to about 15 days;
**g)** Selecting a third sub-set from said second sub-set of strains based on an autotrophic growth rate not higher than 60% of growth rate the parental strain;
**h)** Optionally further subjecting said third subset of strains to a random mutagenesis to obtained a genetically mutated strain set;
**i)** Optionally subjecting said mutated strain set to steps b) to g) to obtain a final sub-set of mutated strains.
**j)** Collecting the isolated strains.

It is advantageous to provide a high-protein microalgae product suitable for human consumption with reduced chlorophyll pigment. The method of the invention advantageously reduces the pigments content without affecting the protein content.

Disclosed herein, according to a further aspect of the invention, are new strains of microalgae belonging to green algae and blue-green algae of *Chlorella* genus, in particular C. *vulgaris.* Disclosed herein, according to a further aspect of the invention, is a high-protein microalgae product suitable for human consumption and containing not more than 2 % w/w chlorophyll content.

Disclosed herein, according to a further aspect of the invention, is a composition for food or a food additive, comprising one or more microalgae strains according to the invention.

The invention is based on the unexpected finding that the method of selecting microalgae according to the invention allows to isolate microalgae strains with interesting physiological profile with nutritional advantages such as being suitable for human consumption. Their low chlorophyll content results in an algal biomass easy to manipulate without problem of chlorophyll degradation which leads to a not suitable smell and color.

Other features and advantages of the invention will be apparent from the claims, detailed description, and figures.

### Description of the figures

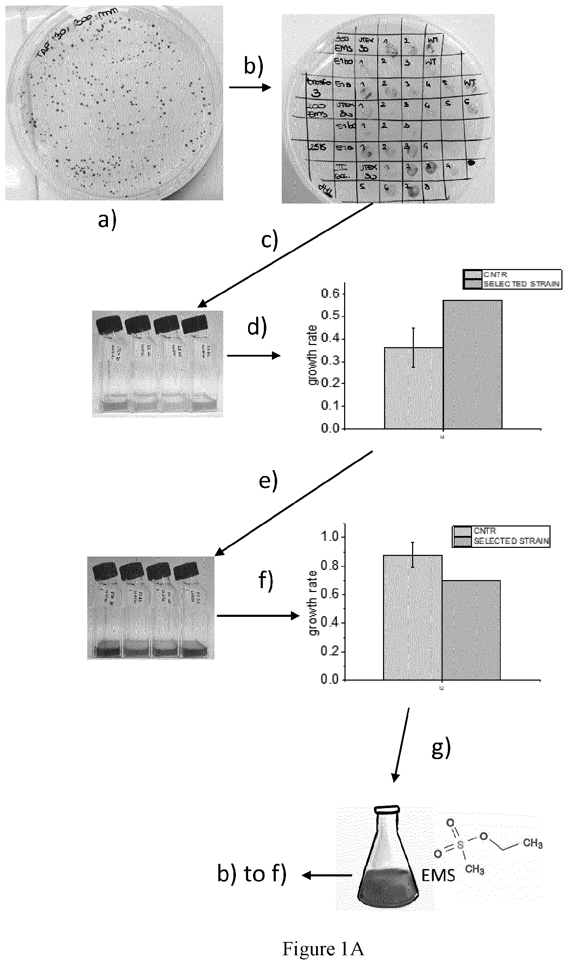

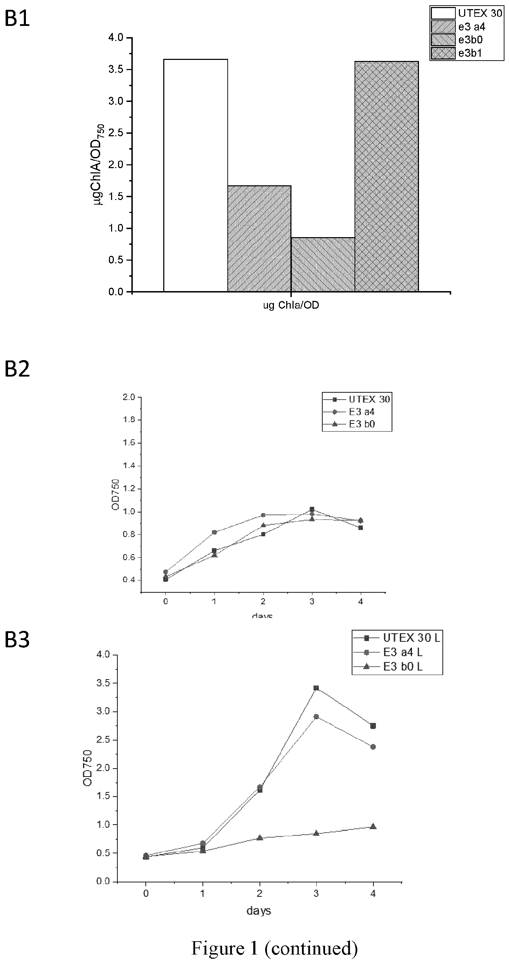

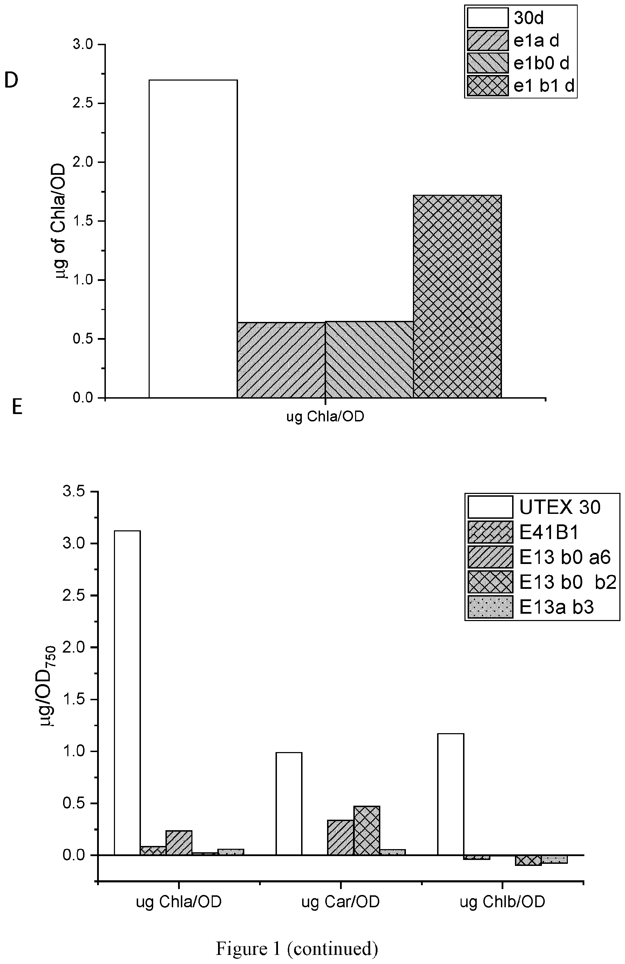

**Figure 1** illustrates steps **a)** to **i)** of a method according to the invention and the resulting selected strains as described in Example 1. **A:** schematic representation of the steps of the method; **B:** illustration of the selection steps of the method; **B1:** selecting a first sub-set of strains based on their reduced coloration based on levels of parental strain **B2:** selecting a second sub-set from said first sub-set of strains based on a heterotrophic growth rate not lower than 80% with respect to the parent strain; **B3:** selecting a third sub-set from said second subset of strains based on an autotrophic growth rate not higher than 60% of the WT; **D:** chlorophyll content of the resulting third sub-set of strains before second round of mutagenesis expressed in micrograms of chlorophyll per unit of optical density at 664 nm; **D:** characterization of the selected strains based on their pigment content in the dark and in light conditions as described in Example 2. L stands for "light" (growth in autotrophic condition).

### Detailed description

"Microalgae" refers to microscopic eukaryotic single cells composed of a nucleus, one or more chloroplasts, mitochondria, Golgi bodies, endoplasmic reticulum and other organelles. Microalgae suitable in the context to the invention belong to the phylum *Chlorophyta,* preferably *Chlorella* genus, more preferably *Chlorella vulgaris.* Those include species such as *Chlorella protothecoides, Chlorella ellipsoidea, Chlorella minutissima, Chlorella zofinienesi, Chlorella luteoviridis, Chlorella kessleri, Chlorella sorokiniana, Chlorella fusca var. vacuolata Chlorella sp., Chlorella cf. minutissima* or *Chlorella emersonii.*

Other species of *Chlorella* can be selected from the group consisting of *anitrata, Antarctica, aureoviridis, candida, capsulate, desiccate, ellipsoidea* (including strain CCAP 211/42), *emersonii, fusca* (including *var. vacuolata), glucotropha, infusionum* (including *var. actophila* and *var. auxenophila), kessleri* (including any of UTEX strains 397,2229,398), *lobophora* (including strain SAG 37.88), *luteoviridis* (including strain SAG 2203 and *var. aureoviridis and lutescens), miniata, cf. minutissima, minutissima* (including UTEX strain 2341), *mutabilis, nocturna, ovalis, parva, photophila, pringsheimii, protothecoides* (including any of UTEX strains 1806, 411 , 264, 256, 255, 250, 249, 31, 29, 25 or CCAP 211/8D, or CCAP 211/17 and *var. acidicola), regularis* (including *var. minima,* and *umbricata), reisiglii* (including strain CCP 11/8), *saccharophila* (including strain CCAP 211/31, CCAP 211/32 and *var. ellipsoidea), salina, simplex, sorokiniana* (including strain SAG 211.40B), *sp.* (including UTEX strain 2068 and CCAP 211/92), *sphaerica, stigmatophora, trebouxioides, vanniellii, vulgaris* (including strains CCAP 211/1 IK, CCAP 211/80 and *f. tertia* and *var. autotrophica, viridis, vulgaris, vulgaris f. tertia, vulgaris f. viridis), xanthella,* and *zofingiensis.*

Therefore, the microalgal material can be non-green such as yellow, or yellow-white (due to the microalgae mutation).

The expression "Chlorophyll" refers to the principal pigment involved in photosynthesis. It allows for the photosynthetic organism to absorb the energy of the light in order to convert CO₂ into carbohydrates necessary for its growth. This pigment can be found in different forms: Chlorophyll a, b, c and d (and sometimes but rarely e). It is formed by a central magnesium atom surrounded by a porphyrin ring (nitrogen containing structure). The content of chlorophyll in microalgae can be determined by different methods, most of them involving the extraction of the pigments for example with organic solvents such as acetone, methanol, ethanol, etc., followed by a quantification using spectrophotometric analysis. Several methods can be found in the literature, such as the method described by Pruvost et al., Bioresource Technology, 102(1), 150-158. doi:10.1016/j.biortech.2010.06.153*.*

The expression "heterotrophic fermentation conditions" refers to conditions for cell growth and propagation using an external carbon source under dark conditions. Several examples of microalgae growth under heterotrophic conditions, using different organic carbon sources, are summarized in the literature *(*Hu et al., 2018, Biotechnology Advances 36, 54-67*).*

A *"microalgae culture medium"* suitable for heterotrophic fermentation conditions comprises **i)** a carbon source and **ii)** nutrients for microalgal growth. According to the present invention, typical heterotrophic fermentation conditions comprise dark at 21°C (and shaking in case of liquid cultures).

Nutrients for microalgae growth suitable for heterotrophic fermentation conditions are known to the skilled person and can be found for example under https://utex.org/pages/algal-culture-media (University of Texas at Austin for its algal culture collection (UTEX)).

The microalgal biomass is itself a finished food ingredient and may be used in foodstuffs without further, or with only minimal, modification. Alternatively, after concentration, microalgal biomass can be processed to produce different food or feed products containing microalgae as an ingredient aiming to increase the nutritional value (pasta, beverages, drinks, meat substitutes, etc.) and/or as food supplements (in form of capsules, pills, etc).

Referring to the figures, in particular first to **Figure 1**, is provided an illustration of a method for the selection of microalgae. The illustrated method for the production of microalgae generally comprises the steps of: providing a plurality of microalgae strains belonging to the same green algae and blue-green algae genus which were obtained by treating the parent strain from the said green algae and blue-green algae genus with an mutagenic agent such as EMS (Ethyl Methane Sulfonate) and plated to a microalgae heterotrophic culture medium (a), growing said plurality of strains in said heterotrophic culture medium under heterotrophic culture conditions (b), selecting a first sub-set of strains based on their reduced coloration (c), subjecting said first sub-set of strains with a reduced coloration to a growing step in microalgae heterotrophic culture medium under heterotrophic culture conditions for about 1-10 days (d), selecting a second sub-set from said first sub-set of strains based on a heterotrophic growth rate not lower than 80% with respect to the growth rate of the parental strain in the same conditions (e), growing said second sub-set under autotrophic culture medium under autotrophic culture conditions for about 1 to 15 days (f), selecting a third subset from said second sub-set of strains based on an autotrophic growth rate not higher than 60% with respect to the growth rate of the parental strain in the same conditions from said first sub-set of strains based on a heterotrophic growth rate not lower than 80% with respect to the growth rate of the parental strain in the same conditions (g). The third subset of strains can then be subjected to further random mutagenesis to obtain a genetically mutated strain set (i) and said mutated strain set is subjected to one or more step sequence b) to g) as previously described to obtain a final sub-set of mutated strains (h).

More specifically, the steps of the embodiment illustrated in Figure 1 comprise the following steps:
a) Providing a plurality of microalgae strains belonging to the same green algae and blue-green algae genus to a microalgae heterotrophic solid culture medium;
b) Growing said plurality of strains in said heterotrophic culture medium under heterotrophic culture conditions for about 10 to about 60 days (e.g. about 30 days);
c) Selecting a first sub-set of strains based on their reduced coloration compared to a control strain (e.g. parental);
d) Subjecting said first sub-set of strains with a reduced coloration to a growing step in microalgae heterotrophic culture medium under heterotrophic culture conditions for about 1 to about 10 days (e.g. about 5 days);
e) Selecting a second sub-set from said first sub-set of strains based on a heterotrophic growth rate not lower than 80% with respect to the parental strain;
f) Growing said second sub-set under autotrophic culture medium under autotrophic culture conditions for about 1 to about 15 days (e.g. about 5 days);
g) Selecting a third sub-set from said second sub-set of strains based on an autotrophic growth rate not higher than 60% with respect to the growth rate of the parental strain in the same conditions;
h) Optionally further subjecting said third subset of strains to a random mutagenesis to obtained a genetically mutated strain set;
i) Optionally subjecting said mutated strain set to steps b) to g) to obtain a final sub-set of mutated strains.
j) Collecting the isolated strains.

According to another particular embodiment, the plurality of microalgae strains under step a) is provided in the form of mutated strains obtained from mutagenesis of a natural strain. According to another particular embodiment, mutagenesis of a natural strain (parental) is obtained by contacting the third sub-set with a mutagenic agent such as EMS (Ethyl Methane Sulfonate).

According to a particular embodiment, the plurality of microalgae strains is provided at a concentration of about 15^{∗}10⁶ cells, divided in 1^{∗}10⁴ cells per plate.

According to a particular embodiment, step b) is carried out until single colonies appear. The appearance of single colonies can be checked by visual inspection where pale and more colored colonies appear.

According to a particular embodiment, step b) is carried by visual observation or by quantifying the chlorophyll fluorescence, e.g. with a fluorescence camera (FluorCam FC 800 video-imaging apparatus (Photon Systems Instruments, Brno, Czech Republic) as described in Perin et al., 2015, Biotechnology for Biofuels, 8:161 DOI 10.1186/s13068-015-0337-5*.*

According to a particular embodiment, step h) is carried out by contacting the third sub-set with a mutagenic agent such as EMS (Ethyl Methane Sulfonate).

According to a particular aspect of the invention, the microalgae is any microalgae suitable for mammal an in particular human consumption.

According to a particular aspect of the invention, the microalgae is from the phylum *Chlorophyta.*

According to a particular aspect of the invention, the microalgae is *Chlorella.*

According to a particular aspect of the invention, the microalgae is *Chlorella vulgaris.* According to a further particular aspect of the invention, the parental strain is *Chlorella vulgaris* UTEX30.

According to another particular aspect of the invention, the method of the invention further comprises one or more step of further selecting a sub-set of strains from said collected and isolated strains under step **j),** said step being selected from **k)** selecting strains based on their lowest chlorophyll content per cell; **1)** selecting strains for their fastest growing rate in nutrient limitation conditions; **m)** selecting strains for their growing yield in a photobioreactor. Typically, a photobioreactor such as described in Dasan et al., 2021, IOP Conf. Series: Earth and Environmental Science 721, 012013 IOP Publishing doi:10.1088/1755-1315/721/1/012013 or in Singh et al., 2012, Development of suitable photobioreactor for algae production-A review, Renewable and Sustainable Energy Reviews, 16(4*)* can be used.

According to a further particular aspect, the chlorophyll content on chlorophyll a is evaluated based on OD at 664 nm and the chlorophyll content on chlorophyll b is evaluated based on OD at 646.8 nm.

According to a particular aspect of the invention, the method of the invention further comprises a drying step of the harvested microalgae material.

Another particular aspect of the invention, is to provide a method or a process for selecting a microalgae material, wherein the said microalgal material contains at least 10% of proteins.

According to another particular aspect of the invention, is provided a strain of microalgae belonging to green algae and blue-green algae of *Chlorella* genus, in particular C. *vulgaris* obtainable from a method according to the invention wherein the chlorophyll content of the strain is below 2% w/w.

According to another particular aspect of the invention, is provided a C. vulgaris strain according to the invention.

According to a further particular aspect of the invention, is provided a microorganism which is, or has the identifying characteristics of, a strain of C. *vulgaris* deposited with the Culture Collection of Algae and Protozoa from SAMS (Scottish Association for Marine Science) under an accession number selected from CCAP 211/137, CCAP 211/138 and CCAP 211/139, or a mutant strain derived therefrom.

According to a further particular aspect of the invention, is provided a microorganism which is, or has the identifying characteristics of, a strain of C. *vulgaris* deposited with the Culture Collection of Algae and Protozoa from SAMS (Scottish Association for Marine Science) under the accession number CCAP 211/137 or a mutant strain derived therefrom.

According to a further particular aspect of the invention, is provided a microorganism which is, or has the identifying characteristics of, a strain of C. *vulgaris* deposited with the Culture Collection of Algae and Protozoa from SAMS (Scottish Association for Marine Science) under the accession number CCAP 211/138 or a mutant strain derived therefrom.

According to a further particular aspect of the invention, is provided a microorganism which is, or has the identifying characteristics of, a strain of C. *vulgaris* deposited with the Culture Collection of Algae and Protozoa from SAMS (Scottish Association for Marine Science) under the accession number CCAP 211/139 or a mutant strain derived therefrom.

According to another particular aspect of the invention, is provided a composition comprising a microalgal material derived from the strain of the invention or a strain obtainable from a method of the invention.

According to another further particular aspect of the invention, the microalgal material derived from a strain of the invention or a strain obtainable from a method of the invention has a chlorophyll content in a range of 3.6 µg of chlorophyll/OD to 0.09 µg of chlorophyll/OD, preferably 1.31 to 0.09 µg of chlorophyll/OD.

According to another particular aspect of the invention, the composition is a food or a feed product or ingredient.

According to another particular aspect of the invention, the composition is a cosmetic product or cosmetic ingredient.

According to another particular aspect of the invention, is provided a use of a strain or a composition according to the invention for the preparation of a food or a feed product. According to another particular aspect of the invention, is provided a food or a feed product comprising a microalgae material according to the invention.

According to another further particular aspect of the invention, is provided a high-protein microalgae product suitable for human or mammalian consumption and containing not more than 2 % w/w chlorophyll content.

The invention is based on the unexpected finding that the method of selecting microalgae according to the invention allows to isolate microalgae strains with interesting physiological profile with nutritional advantages such as being suitable for human consumption. Microalgae strains according to the invention are low chlorophyll content strains, so they exhibit a strong reduction in photosynthesis and they need to be grown in heterotrophy. The reduction in chlorophyll content ensures a microalgae biomass without a bad smell and a brown color, usually due to chlorophyll photooxidation and degradation therefore those are particularly useful for human consumption.

Examples illustrating the invention will be described hereinafter in a more detailed manner and by reference to the embodiments represented in the Figures.

### EXAMPLES

### Example 1: Method of selection of strains according to the invention

The method of the invention was used to isolate strains according the invention as follows. The main steps of the method are illustrated under Figure 1.

### - Providing a plurality of microalgae strains belonging to the same green algae and blue-green algae genus to a microalgae heterotrophic culture medium (step a)

A green algae train was chosen as parental strain based on its protein content (around 30% (w/w)) and its growth rate in heterotrophic conditions (0.36193 d⁻¹): *Chlorella vulgaris* strain UTEX30 (UTEX Culture Collection of Algae, 205 W. 24th St., Biological Labs 218, The University of Texas at Austin (A6700) Austin, TX 78712 USA).

Protein content is measured by Bradford assay *(*Bradford, 1976, Anal Biochem., May 7;72:248-54*),* using acidified Coomassie Brilliant Blue G-250. The binding of the dye to proteins allows a color change in the visible spectrum. The growth rate in heterotrophic condition is evaluated with a growth curve, monitoring daily the optical density at 750 nm. An increase in this parameter is symptomatic of cell duplication and biomass production. Parental strain and mutated strains have been inoculated at the same starting optical density. Acetate was added to the medium to provide a carbon source for growth in the dark.

Mutations were performed on the parental strain as described below to lead to a plurality of mutated microalgae strains belonging to the same green algae and blue-green algae genus. The starting material are parent strain cells grown for 5 days in dark Tris-acetate-phosphate medium (TAP) medium starting from OD at 750 nm: 0.2. Then, 15^{∗}10⁶ cells (1^{∗}10⁴ cells per plate, considering the 90% of mortality, this correspond to 1'000 colony per plate) from the parent strain resuspended in 500 µ1 of Tris-acetate-phosphate medium (TAP) medium are treated with a mutagen 300 mM EMS (Ethyl Methane sulfonate).

### - Growing said plurality of strains in said heterotrophic culture medium under heterotrophic culture conditions for about 1 hour (step b)

The plurality of strains obtained *in situ* by mutagenesis is incubated for about 1 hour in the dark to ensure the mutagen action. Then, the plates were washed with 1 ml of TAP combined with Kanamycin (KAN) (50 µg/ml) and Ampicillin (Amp) (100 µg/ml) (5'000g 8 min), followed by 1 h of recovery in TAP + Kan+ Amp **(****Fig. 1Aa****).** Approximately 3 weeks after mutagenesis, single colonies appeared, and they were collected in a plate in order to have a spot from each colony (**Fig. 1Ab**).

### - Selecting a first sub-set of strains based on their reduced coloration compared to a control strain (e.g. parental) (step c)

A first sub-set of strains was selected based on their reduced coloration. This selection can be made by visual observation. 16 strains were isolated in a first sub-set of strains. As can be seen from **Fig. 1B1**, the chlorophyll content of strains **E3a4** is only 43% of the parental line; **E3b0,** 23%, while for **E3b1,** it is equivalent to parental line. Therefore, **E3b1** was discarded from the first subset.

### - Subjecting said first sub-set of strains with a reduced coloration to a growing step in microalgae heterotrophic culture medium under heterotrophic culture conditions for about 5 days (step d)

The first subset of strains was incubated for about 5 days in the dark under heterotrophic culture conditions as detailed above. The growth rate in heterotrophic condition has been evaluated with a growth curve. The growth has been measured considering the optical density at 750 nm. An increase in this parameter is symptomatic of cell duplication and biomass production. Parental strain and pale strains have been inoculated at the same optical density in dark (0.2). To ensure the carbon source acetate was added. The growth has been monitored for 5 days.

### - Selecting a second sub-set from said first sub-set of strains based on a heterotrophic growth rate not lower than 80% of the growth of the parent under the same conditions (step e)

As can be seen on **Figure 1B2**, strains **E3a4** and **E3b0** which exhibit lower chlorophyll contents than the control strain (parental UTEX 30) all have a growth curve comparable to the growth curve of parental strain (UTEX30) and therefore, those were selected in a second subset of 14 strains.

### - Growing said second sub-set under autotrophic culture medium under autotrophic culture conditions for about 5 days (step ƒ)

The second subset of strains was incubated for about 5 days in the light under autotrophic culture conditions. The procedure is the same described for dark conditions, just with the exposure of the culture at 150 µmol of photons m⁻²s⁻¹, instead of dark.

### - Selecting a third sub-set from said second sub-set of strains based on a heterotrophic growth rate not higher than 60% of the growth of the parent under the same conditions (step g)

As can be seen on **Figure 1B3**, strain **E3b0** show less growth than the parental strain (UTEX30) and was thus retained. E3a4 was instead discarded.

### - Subjecting said third subset of strains to mutagenesis to obtained a genetically mutated strain set (step h)

The third subset was composed of 3 strains **(elad, elbo d, e1 b1d)** which have been selected for all criteria from steps b) to g) of the screening method (**Fig. 1D**) were then subjected to mutagenesis as described above to obtain further mutated strains.

### - Subjecting said mutated strain set to steps b) to h) to obtain a final sub-set of mutated strains (step i)

The obtained 3 mutated strains were subjected to a 2^{nd} round of steps b) to g) to lead to a final sub-set of about 6'0000 mutated strains.

### Said mutated strain tested in nutrient depletion condition to isolate the fastest

The 3 isolated mutants after the third set of screening have been subjected to a growth test to select the fasted growing in limited nutrient condition (half nutrient concentration). 2 strains were then finally selected.

### Selected strains tested in bioreactor

The selected strains have been tested in a bioreactor to check protein content and chlorophyll content.

A summary of the final sub-set of mutated strains is provided under **Table 1** below.

**Table 1**

| **Strain** | **Parent strain** | **Growth curve (dark)** | **Growth curve (light)** | **Chl. Content (dark)** | **Chl/carotene** | **Chl a/b** | **Protein content** |
|---|---|---|---|---|---|---|---|
| E1_3b0 a.6 | E1b.O | ≈WT | 0 | 5% | ≈E1b0 in Dark, >Chl in light | ≈E1b0 | ≈WT |
| E1 3b0 b.2 | E1b.O | ≈WT | 0 | 12% | <Chl in dark | > Chla | ≈WT |
| E4 1b1 | E1b.1 | ≈WT | 0 | 2% | | | ≈WT |

| | | | | | | | |
|---|---|---|---|---|---|---|---|
| *^{∗}E1b0 is deposited under accession number CCAP 211*/*138.* | | | | | | | |

### Example 3: Characterization of the selected strains

The 3 selected strains obtained in Example 1 were further characterized as follows in terms of pigment content under dark and light conditions.

For this purpose, chlorophylls (a and b) and carotenoids were extracted by incubating the pellet obtained from 1 mL of a strain culture of the strains from the final subset of mutated strains obtained under Example 2 with DMF (dimethylformamide) overnight at 4°C in the dark. Pigments content was quantified from the extract due to the solubilization of cells with DMF using a spectrophotometer a Cary 100 spectrophotometer (Agilent Technologies, California, USA) using specific extinction coefficients Wellburn 1994, Plant physiology, 144, 307-13*).*

**Fig. IE** represents the quantification of the pigments of various strains listed in Table 1 above. Among those, **E1_3b0 a.6, E4_1b1** (deposited under accession number CCAP 211/137, aslso called **E14b1, E1 3b0 b.2** (deposited under accession number CCAP 211/139, also called **E2b0)** strains were selected.

## Claims

1. A method for the selection of microalgae, wherein said method comprises the following steps:
a. Providing a plurality of microalgae strains belonging to the same green algae and blue-green algae genus to a microalgae heterotrophic culture solid medium;
b. Growing said plurality of strains in said heterotrophic culture medium under heterotrophic culture conditions for about 10 to about 60 days;
c. Selecting a first sub-set of strains based on their reduced coloration;
d. Subjecting said first sub-set of strains with a reduced coloration to a growing step in microalgae heterotrophic culture medium under heterotrophic culture conditions for about 1 to about 10 days;
e. Selecting a second sub-set from said first sub-set of strains based on a heterotrophic growth rate not lower than 80% of the growth rate of the parental strain under the same conditions;
f*.* Growing said second sub-set under autotrophic culture medium under autotrophic culture conditions for about 1 to about 15 days;
g. Selecting a third sub-set from said second sub-set of strains based on an autotrophic growth rate not higher than 60% of the growth rate of the parental strain under the same conditions;
h. Optionally further subjecting said third subset of strains to a random mutagenesis to obtained a genetically mutated strain set;
i. Optionally subjecting said mutated strain set to steps b) to g) to obtain a final sub-set of mutated strains;
j. Collecting the isolated strains.

2. A method according to claim 2 wherein the plurality of microalgae strains is provided at a concentration of about 15^{∗}10⁶ cells, divided in 1^{∗}10⁴ cells per plate.

3. A method according to any one of the preceding claims wherein step b) is carried out until single colonies appear.

4. A method according to any one of the preceding claims wherein the microalgae is from the phylum *Chlorophyta, in particular Chlorella such as Chlorella vulgaris.*

5. A method according to any one of the preceding claims wherein the isolated strains have a chlorophyll content in a range of 3.6 µg of chlorophyll/OD to 0.09 µg of chlorophyll/OD, preferably 1.31 to 0.09 µg of chlorophyll/OD.

6. A method according to any one of the preceding claims wherein step h) is carried out by contacting the third sub-set with a mutagenic agent such as EMS (Ethyl Methane Sulfonate).

7. A method according to any one of the preceding claims wherein the parental strain is *Chlorella vulgaris* UTEX30.

8. A method according to any one of the preceding claims wherein said method further comprises a drying step of the harvested microalgae material.

9. A strain of microalgae belonging to green algae and blue-green algae of *Chrorella* genus, in particular C. *vulgaris* obtainable from a method according to any one of claims 1 to 5 wherein the chlorophyll content of the strain is below 2% w/w.

10. A microorganism which is, or has the identifying characteristics of, a strain of C. *vulgaris* deposited with the Culture Collection of Algae and Protozoa from SAMS (Scottish Association for Marine Science) under an accession number selected from CCAP 211/137, CCAP 211/138 and CCAP 211/139, or a mutant strain derived therefrom.

11. Use of a strain according to any one of claims 8 to 10 for the preparation of a food or a feed product.

12. A composition comprising a microalgal material derived from the strain according to any one of claims 8 to 10 or a strain obtainable from a method according to any one of claims 1 to 7.

13. A composition according to claim 12, wherein said composition is a food or a feed product or ingredient.

14. A composition according to claim 12, wherein said composition is a cosmetic product or cosmetic ingredient.

15. A food or a feed product comprising a microalgae material derived from the strain according to any one of claims 9 to 10 or a strain obtainable from a method according to any one of claims 1 to 7.
